# EUROPEAN PATENT APPLICATION

(11) **EP 3 249 005 A1**
(43) Date of publication of application: **29.11.2017**
(21) Application number: 16171005.8
(22) Date of filing: 24.05.2016
(51) Int. Cl.: C08K 5/06, C08K 5/14, C07C 409/00, C08K 5/00

(54) **PEROXIDE FORMULATION**

(71) Applicant: Akzo Nobel Chemicals International B.V., 6824 BM Arnhem (NL)
(72) Inventor: TAMMER, Marinus Catharinus, NL-7431 ZA Diepenveen (NL)
(74) Representative: Akzo Nobel IP Department

(57) **Abstract**

Peroxide formulation comprising a peroxide and phenyl tert-butyl ether and its use for producing, modifying, curing, and crosslinking polymers and resins.

## Description

The invention relates to a peroxide formulation comprising a peroxide and a phlegmatizer.

As is well known, organic peroxides are thermally labile compounds. Because the decomposition of these peroxides is exothermic, it is hazardous when the heat of decomposition cannot be dissipated, e.g., by heat loss to the surrounding area. When heat accumulates, the decomposition reaction may run out of control. To avoid such undesired situation, the peroxide is typically diluted with one or more phlegmatizers.

Ketone peroxides, such as methyl ethyl ketone peroxide (MEKP) and methyl isopropyl ketone peroxide (MiPKP), are known as curing agents for unsaturated polyester resins. A ketone peroxide is a reaction product of a ketone and hydrogen peroxide and usually is a mixture of products with different structure types, as described in, for instance, WO 2002/053531.
The preferred phlegmatizers for ketone peroxides are dimethyl phthalate, diisononylphthalate, and 2,2,4-trimethyl-1,3-pentanediol-diisobutyrate (TXIB).
Formulations in dimethyl phthalate and di-isononylphthalate are very stable, in terms of active oxygen content. However, due to legal provisions and growing environmental awareness and perceptions, producers are increasingly forced to use non-phthalate plasticizers. For instance, today's legislation does not any longer allow certain phthalates to be present in polymers that come into contact with food products. In general, phthalates have a bad reputation and will be phased out.
Formulations in TXIB, on the other hand, are less stable than in dimethyl phthalate.

Therefore, there is a desire for providing peroxide formulations, more in particular ketone peroxide formulations, with a stability comparable to that of formulations comprising dimethyl phthalate, but without the legislative and environmental concerns of phthalates.

This object has been met by the present invention, which uses phenyl tert-butyl ether as the phlegmatizer. The present invention therefore relates to a peroxide formulation comprising a peroxide and phenyl tert-butyl ether.

The peroxide formulation according to the present invention is preferably a homogeneous liquid, such as a solution of the peroxide in the phlegmatizer or a homogeneous mixture of a liquid peroxide and the phlegmatizer. In other words, the peroxide formulation preferably does not have the form of a suspension of emulsion.

The peroxide can be selected from the group consisting of ketone peroxides, diacyl peroxides, peroxyesters, peroxyketals, cyclic ketone peroxides, peroxycarbonates, peroxydicarbonates, dialkyl peroxides, and hydroperoxides. The peroxide is preferably liquid at room temperature.
In a preferred embodiment, the peroxide is selected from the group consisting of ketone peroxides, cyclic ketone peroxides, peroxyesters, diacyl peroxides, peroxydicarbonates, and hydroperoxides. More preferably, the peroxide is a ketone peroxide. Most preferably, it is selected from the group consisting of methyl ethyl ketone peroxide, methyl isobutyl ketone peroxide, methyl isopropyl ketone peroxide, acetylacetone ketone peroxide, cyclohexanone ketone peroxide, and methyl isoamylketone peroxide
Alternatively, the peroxide can be a mixture of two or more peroxides. It is preferred that one of the peroxides present in said mixture is a ketone peroxide, more preferably methyl ethyl ketone peroxide. The one or more additional peroxide(s) is preferably a hydroperoxide, more preferably selected from hydrogen peroxide, tert-butyl hydroperoxide, and cumyl hydroperoxide.

Ketone peroxide formulations may contain (residual) hydrogen peroxide. It was observed that in certain curing processes of unsaturated polyesters the speed of gelation was increased due to the presence of the H₂O₂. However, in some instances, such as when vinylester resins are cured, the presence of H₂O₂ is undesirable as it gives foaming. Therefore, depending on the use of the formulations, it can be preferred to have from 0.001 to 5% by weight (%w/w) of hydrogen peroxide in the formulation.

The total concentration of phenyl tert-butyl ether in the formulation is preferably in the range 2-95 wt%, more preferably 5-85 wt%, and most preferably 15-75 wt%.

The total concentration of peroxide in the formulation is preferably in the range 1-95 wt%, more preferably 5-85 wt%, even more preferably 15-75 wt%, and most preferably 20-60 wt%.

In addition to phenyl tert-butyl ether, an additional phlegmatizer (a cophlegmatizer) may be present. Such co-phlegmatizer can be present in an amount of 1-75 wt%, more preferably 2-65 wt%, and most preferably 3-45 wt%. These co-phlegmatizers preferably belong to the group of ethers, esters, alcohols or alkanes. Examples of co-phlegmatizers are diacetone alcohol, glycols, isododecane, and distillation fractions with C10 to C20 alkane mixtures, such as Spirdane D60.

The chemical stability of the formulation can, in some instances, be even further improved by the addition of one or more known additives including sequestering agents such as dipicolinic acid and/or antioxidants such as 2,6-di(t-butyl)-4-methyl phenol and para-nonyl phenol.

The formulation of the present invention may also contain optional other additives as long as these additives do not have a significant negative effect on the transportability and/or storage stability of the formulations. As examples of such additives may be mentioned: anti-caking agents, free-flowing agents, anti-ozonants, antioxidants, anti-degradants, U.V. stabilizers, coagents, fungicides, antistats, pigments, dyes, coupling agents, dispersing aids, blowing agents, lubricants, process oils and mould-release agents. These additives may be employed in their usual amounts.
The formulation of the present invention can be prepared by simply mixing the peroxide and phenyl tert-butyl ether (and the optional additional compounds) or by producing the peroxide in a phenyl tert-butyl ether solution.

The formulation of the present invention is useful for curing unsaturated polyesters, acrylics, and vinyl resins, for polymerizing radically polymerizable monomers (e.g styrene, acrylic monomers, vinyl chloride, and ethylene), and in (co)polymer modification processes for the cross-linking, degradation or other types of modification of (co)polymers.

## Claims

1. Peroxide formulation comprising a peroxide and phenyl tert-butyl ether.

2. Peroxide formulation according to claim 1 wherein the phenyl tert-butyl ether is present in the formulation in an amount of 2-95wt%.

3. Peroxide formulation according to claim 2 wherein phenyl tert-butyl ether is present in the formulation in an amount of 5-85 wt%.

4. Peroxide formulation according to claim 3 wherein phenyl tert-butyl ether is present in the formulation in an amount of 15-75 wt%

5. Peroxide formulation according to any one of the preceding claims wherein the peroxide is selected from the group consisting of cyclic ketone peroxides, ketone peroxides, peroxyesters, diacyl peroxides, peroxydicarbonates, and hydroperoxides.

6. Peroxide formulation according to claim 5 wherein the peroxide is a ketone peroxide.

7. Peroxide formulation according to claim 6 wherein the ketone peroxide is selected from the group consisting of methyl ethyl ketone peroxide, methyl isopropyl ketone peroxide, isobutyl ketone peroxide, acetylacetone ketone peroxide, cyclohexanone ketone peroxide, and methyl isoamylketone peroxide.

8. Peroxide formulation according to claim 6 or 7 comprising up to 5% by weight of hydrogen peroxide.

9. Process for curing an unsaturated polyester, an acrylic resin, or a vinyl ester resin wherein a peroxide formulation according to any one of the preceding claims is used.

10. Process for crosslinking a polymer wherein a peroxide formulation according to any one of claims 1-8 is used.

11. Process for polymerizing a radically polymerizable monomer wherein a peroxide formulation according to any one of claims 1-8 is used.

12. Process for changing the rheology of a polymer wherein a peroxide formulation according to any one of claims 1-8 is used.
